# EUROPEAN PATENT SPECIFICATION

(11) **EP 4 284 938 B1**
(45) Date of publication and mention of the grant of the patent: **22.04.2026**
(21) Application number: 22746522.6
(22) Date of filing: 26.01.2022
(51) Int. Cl.: C12P 7/649, C11C 3/10, C10L 1/19, C10G 3/00, C12N 9/78

(54) **METHODS OF PRODUCING LOW CLOUD POINT BIODIESEL FROM COCOA BUTTER**
VERFAHREN ZUR HERSTELLUNG VON BIODIESEL MIT NIEDRIGEM TRÜBUNGSPUNKT AUS KAKAOBUTTER
PROCÉDÉS DE PRODUCTION D'UN BIODIESEL À BAS POINT DE TROUBLE À PARTIR DE BEURRE DE CACAO

(30) Priority: 28.01.2021 US 202163142620 P
(43) Date of publication of application: 06.12.2023
(73) Proprietor: RHI & Kristian LLC, Longmont, Colorado 80503 (US)
(72) Inventor: MOLEY, Kristian Rene Yehl, Longmont, Colorado 80503 (US); O'DONNELL, Rhiannon, Longmont, Colorado 80503 (US)
(74) Representative: Fulton, David James
(86) International application number: PCT/US2022/013878
(87) International publication number: WO 2022/164888

(56) References cited:
- EP-B1- 2 496 546
- WO-A1-2007/071046
- WO-A1-2015/069129
- GB-A- 2 091 286

## Description

### TECHNICAL FIELD

The present disclosure relates to methods of producing biodiesel from cocoa butter, and more specifically to methods of producing biodiesel from cocoa butter in which the cloud point of the resultant biodiesel is vastly improved as compared to previously known biodiesels.

### BACKGROUND

A common problem found in traditional diesel fuels is their inability to run in freezing conditions. Depending on the quality and sourcing, petroleum diesel begins to gel when temperatures hover around 10-15°F (-12 to -9°C) as components of the fuel begin to crystalize. Some lower quality diesels have been shown to crystallize in conditions up to 25°F (-4°C). Gelling starts to occur and as a result can clog the fuel system of a vehicle, making it difficult for diesel engines to run in colder environments, especially in the winter season.

Often referred to as "winter diesel," Diesel #1 performs in cold temperatures better than Diesel #2. However, Diesel #1 is more expensive than Diesel #2, and in summer months or warm climates, Diesel #1 may become too thin for proper and/or efficient engine operation. Diesel #1 also has a higher volatility than Diesel #2 due to higher levels of cetane. While higher volatility translates to a faster and more fuel-efficient start, lower volatility means a slower burn while driving, which means better fuel efficiency. Due to the various pros and cons associated with Diesel #1 and Diesel #2, it is common practice to blend the two types of diesels in different ratios depending on the conditions a driver will be travelling in, in order to maintain benefits of each.

Biofuels and biodiesels are preferable to petroleum-based diesels for many reasons, foremost of which is they are more environmentally friendly and sustainable then petroleum-based diesel fuels. However, biodiesels often run into the same issue as petroleum diesels regarding colder road running conditions. For example, biodiesel produced by typical methods suffers from a crystallization/solidification phenomenon when temperatures decrease. Although this crystallization/solidification phenomenon is not limited to biodiesel, the temperature at which biodiesel begins to crystallize/solidify is substantially higher in many biodiesels than in petroleum-based diesel fuel. The crystallized constituents can clog fuel systems in diesel engines using biodiesel and thereby cut off the fuel supply to the engine. In many biodiesels, it is the saturated fatty acids present in the biodiesel that solidify at colder temperatures, with saturated fatty acids generally solidifying at higher temperatures than unsaturated fatty acids.

The temperature at which biodiesel constituents, such as fatty acid methyl esters, begin to precipitate is referred to as cloud point (CP). To lower the temperature at which crystallization occurs and thereby lower the CP of the fuel, several techniques can be used, examples of which include blending biodiesel with petroleum-based diesel, and introducing additives. However, each of these techniques has drawbacks. Blending biodiesel with petroleum-based diesel means that there is a continued reliance on fossil fuels and the negative environmental impacts associated therewith, such as nitrogen oxides and sulfur dioxide emissions, which can create acid rain. Additives are expensive, contain registered carcinogens, and add to the list of emissions and environmental pollutants expelled from an engine exhaust. And while winterization can lower cloud point through a process wherein unsaturated fatty acids are crystallized and removed from the source materials to thereby lower the CP of the resultant biodiesel, the CP of such biodiesel may still be too high for broad commercial use at a wide range of temperature conditions.

Further complicating the production of biodiesel capable of being used at low temperatures is the fact that the selected feedstock of a biodiesel fuel also has a significant impact on the CP of the resulting biodiesel. For example, some potential feedstocks have been deemed unsuitable for use in the production of biodiesel because the CP of biodiesel formed from these feedstocks is too high and therefore may not be viable on-road fuel options. Of particular note, cocoa butter has generally been deemed unsuitable as a legitimate feedstock for the production of biodiesel because biodiesel produced from cocoa butter has a CP of 59 to 62°F (15 to 17°C) when no cold stabilization techniques are employed during production. Even when cold stabilization techniques are employed, the CP of biodiesel produced from cocoa butter is only reduced to 39°F (4°C).

Accordingly, a need exists for both producing a biodiesel having a suitably low cloud point such that the biodiesel can be employed as a viable fuel option and used reliably at a broad range of operating temperatures, and for producing such a low cloud point biodiesel from feedstocks that while being available in large quantities as waste material and which can produce an efficient fuel with reduced emissions, have previously been deemed unsuitable in the production of biodiesel.

EP2496546 relates to a method for lowering cloud point of the fatty acid esters. WO2007/071046 relates to a process for preparing biodiesel.

### SUMMARY

Described herein are various aspects and embodiments of methods for producing low cloud point biodiesel from cocoa butter.

The method for producing low cloud point biodiesel from cocoa butter according to the invention includes the steps of: desaturating cocoa butter using a fatty acid desaturase to thereby produce a desaturated cocoa butter; transesterifying the desaturated cocoa butter to thereby produce biodiesel; performing urea clathration on the biodiesel to thereby form clathrates in the biodiesel; and separating the clathrates from the biodiesel.

### BRIEF DESCRIPTION OF THE DRAWINGS

Many aspects of the present disclosure can be better understood with reference to the following drawings. The components in the drawing are not necessarily to scale. Instead, emphasis is placed on illustrating clearly the principles of the present disclosure. The drawings should not be taken to limit the disclosure to the specific embodiments depicted but are for explanation and understanding only.
Fig. 1 is a flow chart illustrating a method of producing low cloud point biodiesel from cocoa butter according to various embodiments described herein.
Fig. 2 is a flow chart illustrating a method of preparing cocoa butter for conversion to biodiesel.
Fig. 3 is a flow chart illustrating a method preparing cocoa butter for conversion to biodiesel.

### DETAILED DESCRIPTION

Methods of producing a biodiesel derived from cocoa butter and in which the biodiesel possesses a cloud point as low as -61°F (-52°C) are described herein. The methods of producing the biodiesel include a two-step cold stabilization process in which at least two separate processes primarily aimed at reducing the cloud point of the resultant biodiesel are carried out. One of the two cold stabilization steps involves desaturating the cocoa butter prior to transesterifying the cocoa butter to form biodiesel. Desaturating the cocoa butter generally results in the conversion of saturated fatty acids in the cocoa butter to unsaturated fatty acids. As a result, the amount of saturated fatty acids in the cocoa butter is reduced, which helps to lower the cloud point of biodiesel subsequently formed from the desaturated cocoa butter. Furthermore, the step of desaturating the cocoa butter includes converting the cocoa butter from being a fat (and therefore a solid) at room temperature to being an oil (and therefore a liquid) at room temperature. This further conditions the cocoa butter for use as a suitable source material in preparing a low cloud point biodiesel. Once desaturation has taken place via the first step of the two-step cold stabilization process, the cocoa butter is converted to biodiesel using transesterification. The second step of the two-step cold stabilization is then carried out, and employs urea clathration,
which results in the formation of a lattice-like structure of urea having saturated fatty acid methyl ester molecules trapped therein, and that can easily be removed from the biodiesel. As a result, the amount of unsaturated fatty acids in the biodiesel is reduced and the cloud point of the biodiesel is correspondingly lowered.

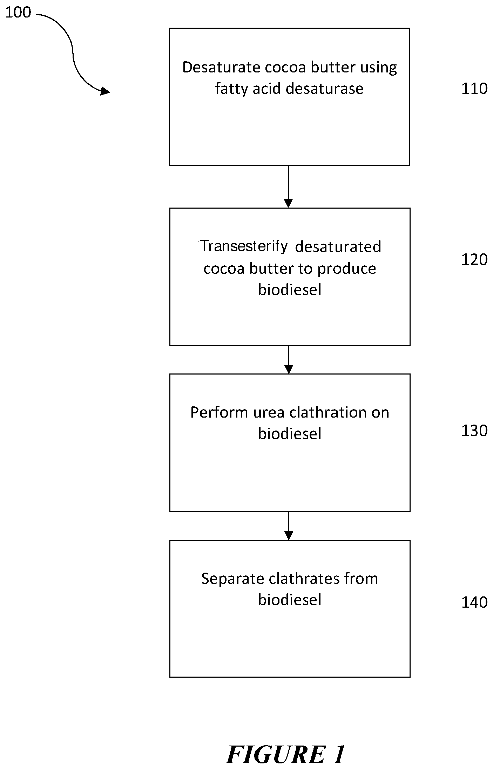

With respect to Fig. 1, a method 100 for producing low cloud point biodiesel from cocoa butter comprises a step 110 of desaturating cocoa butter using a fatty acid desaturase to thereby produce a desaturated cocoa butter, a step 120 of transesterifying the desaturated cocoa butter to thereby produce biodiesel, a step 130 of performing urea clathration on the biodiesel to thereby form clathrates in the biodiesel, and a step 140 of separating the clathrates from the biodiesel.

In step 110, cocoa butter is desaturated in order to reduce the amount of saturated fatty acids present in the cocoa butter. The source and/or type of cocoa butter subjected to desaturation in step 110 is generally not limited. In some embodiments, the cocoa butter is waste byproduct recycled from another process, such as cocoa butter used in the production of chocolate. For example, cocoa butter is typically used to clean machinery used in the production of chocolate, such as to clean machines between flavor or batch changes. Once used in this manner, the cocoa butter is typically treated as water material, but can be used as the feedstock for the production of biodiesel as described herein. The cocoa butter subjected to desaturation in step 110 can be purified or unpurified, though in some embodiments purified cocoa butter is preferred as the cocoa butter subjected to desaturation. Purification of cocoa butter can generally be carried out by, e.g., filtration of impurities from the cocoa butter. As noted previously, the cocoa butter used in step 110 will generally be in the form of a solid fat at room temperature, and as discussed in greater detail below, may therefore need to be melted and converted to a less viscous/more liquid material as part of the desaturation process of step 110. Any suitable amount of cocoa butter can be supplied in step 110, with the ratio of desaturase used being adjusted based on the amount.

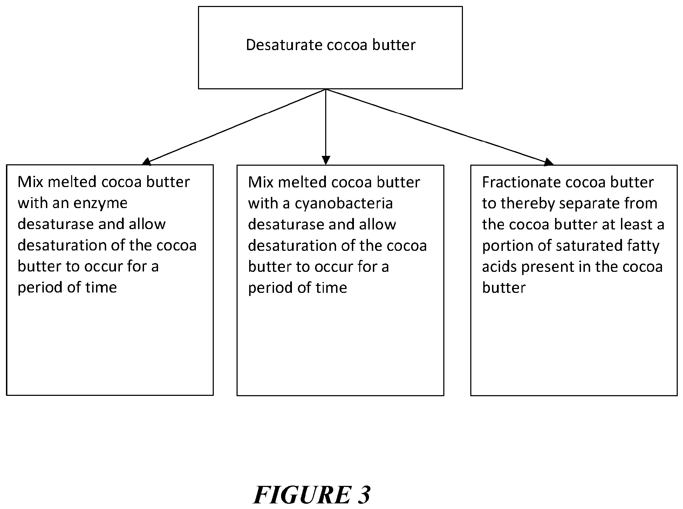

In step 110, the manner of desaturating the cocoa butter includes the incorporation of a fatty acid desaturase into the cocoa butter, where the desaturase works to convert saturated fatty acid components of the cocoa butter (such as saturated fatty acid esters) into unsaturated fatty acids. However, as described in greater detail below with respect to, e.g., Fig. 3, other methods, not according to the invention, of desaturating the cocoa butter can also be used, such as fractionation aimed at separating from the cocoa butter saturated and/or unsaturated fatty acids from the cocoa butter.

In step 110, for the purpose of catalyzing desaturation of the cocoa butter, any suitable desaturase can be used. That is to say, any desaturase capable of being added to cocoa butter for the purpose of converting saturated fatty acids to unsaturated fatty acids can be used. In some embodiments, the desaturates used in step 110 is either an enzyme or a cyanobacteria.

When an enzyme is used as the desaturase in step 110, the enzyme may be any protein that is compatible with the cocoa butter and which facilitates and/or accelerates the conversion of saturated fatty acids to unsaturated fatty acids, typically by acting to catalyze the conversion in which single bonds at specific locations in the fatty acids are converted to double bonds (such as by removal of two hydrogen atoms). In some embodiments, the enzyme desaturase is classified as Δ, indicating that the double bond is created at a fixed position from the carboxyl end of a fatty acid chain. The substrates for the enzymes suitable for use as a desaturase in step 110 may be either acyl-CoA or acyl residues attached to phospholipids. In some embodiments, the enzyme used in step 110 for the purpose of desaturating the cocoa butter is cytochrome b5.

When a cyanobacteria is used, the acyl-lipid desaturases of the cyanobacteria may carry out the desired cocoa butter desaturation. The cyanobacteria may be any cyanobacteria that is compatible with the cocoa butter and which facilitates and/or accelerates the conversion of saturated fatty acids to unsaturated fatty acids, typically by a mechanism in which single bonds at specific locations in the fatty acids are converted to double bonds. In some embodiments, the cyanobacteria used in step 110 for the purpose of desaturating the cocoa butter is a group 3y cyanobacteria, such as *Arthrospira* (a species of *Spirulina*) or *Synechocystis.* For example, with respect to *Synechocystis,* this cyanobacteria catalyzes desaturation at the Δ9, Δ12, Δ6 and ω3 positions of fatty acids that are esterified at the sn-1 position of the glycerol moiety of glycerolipids. Cocoa butter contains a high proportion of saturated fats as well as monounsaturated oleic acid. The Δ9 desaturase introduces the first unsaturated bond into stearic acid to produce oleic acid, which is further desaturated to linoleic acid by the Δ12 desaturase. The Δ6 and ω3 desaturases then introduce unsaturated bonds to generate tri- and tetra-unsaturated fatty acids. Other suitable cyanobacteria that can be used in step 110 include.

The amount of desaturase added to the cocoa butter in step 110 is generally not limited, and may depend on a variety of factors, including but not limited to, the amount of cocoa butter to be desaturated, the specific desaturase being used, and the amount of desaturation desired.

While the above embodiments generally describe the use of a single desaturase in carrying out step 110 of method 100, it should be appreciated that the methods described herein can be modified such that multiple desaturases are used in step 110. This may apply to both the use of more than one class of desaturase in step 110 as well as the use of more than one species of desaturase in step 110. For example, step 110 can be carried out such that both an enzyme desaturase and a cyanobacteria are used in step 100, and/or such that multiple types of enzymes or multiple types of cyanobacteria are used in step 110. When more than one class and/or species of desaturase is used in step 110, the ratio of different desaturases used is not limited, and may be adjusted on any number of factors, including experimentation showing that certain ratios of different classes or species of desaturase improve the overall desaturation (i.e., increase the amount of saturated fatty acids converted to unsaturated fatty acids.

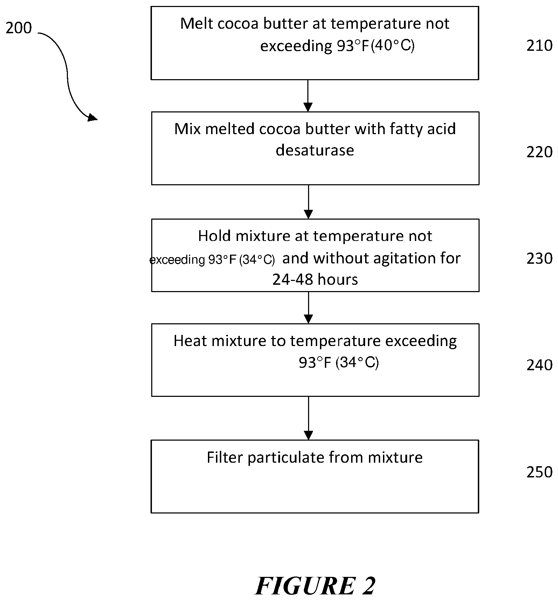

With reference to Fig. 2, a method 200 of preparing cocoa butter for conversion to biodiesel provides additional details regarding step 110 of method 100 illustrated in Fig. 1. Method 200, which generally relates to steps carried out in desaturating cocoa butter, includes step 210 of melting cocoa butter at a temperature not exceeding about 93°F (34°C), a step 220 of mixing the melted cocoa butter with a fatty acid desaturase to thereby form a mixture, a step 230 of holding the mixture at a temperature not exceeding 93°F (34°C) and without agitation for a period of time in the range of from 24 to 48 hours, a step 240 of heating the mixture to a temperature above about 93°F (34°C) and a step 250 of filtering the mixture to remove particulate. The specific steps of method 200 provide information on how to effectively desaturate cocoa butter using a desaturase, taking into account, for example, the conditions under which the desaturase can effectively desaturate cocoa butter and sufficient desaturation has occurred.

In step 210, the cocoa butter is melted by heating the cocoa butter to a temperature that does not exceed about 93°F (34°C). As noted previously, cocoa butter is generally in the form of a solid fat at room temperature, which makes it difficult to mix the cocoa butter with other constituents (such as a desaturase) and to subsequently process the cocoa butter into a biodiesel (via, e.g., transesterification). Heating the cocoa butter to an elevated temperature, such as close to but not exceeding 93°F (34°C), converts the cocoa butter from a solid fat to a liquid oil, thereby making subsequent processing easier. In some embodiments, the cocoa butter is heated to about 93°F (34°C). Any manner of heating the cocoa butter may be used. Generally speaking, the method and rate at which the cocoa butter is heated should be carried out in a manner that avoids scalding or burning the cocoa butter.

As noted previously, step 210 is carried out such that the cocoa butter does not exceed 93°F (34°C). This limitation is aimed at avoiding a situation in which subsequent addition of the desaturase leads to diminishing and/or destroying the integrity of the desaturase due to the melted cocoa butter being at an excessively high temperature. In other words, the temperature of the cocoa butter must be maintained at a level that remains hospitable for the desaturase such that the desaturase is able to properly function when mixed with the cocoa butter (i.e., function to convert saturated fatty acids to unsaturated fatty acids).

Once melted, the cocoa butter is combined and mixed with the desaturase in step 220. Any manner of combining the cocoa butter and the desaturase can be used, with the desaturase generally being added to the cocoa butter based on the mass of the cocoa butter being greater than the desaturase used. Once combined, the cocoa butter and desaturase are gently mixed for a period of time. The period of time for mixing the materials is generally not limited. In some embodiments, the period of time for mixing is in the range of about 15 to 20 minutes, though the amount of mixing can be adjusted based on a variety of factors, including the amount of melted cocoa butter, the consistency of the melted cocoa butter, and other similar considerations. Any period of time of mixing that results in the desaturase being intimately mixed through the cocoa butter can be used in step 220.

Mixing step 220 is generally carried out in the absence of any additional heat being added to the mixture. This is again aimed at avoiding the cocoa butter being heated to a temperature above which the desaturase can effectively function and/or at which point the integrity of the desaturase is compromised or destroyed.

In step 230, the mixture of cocoa butter and desaturase is held at a temperature not exceeding 93°F (34°C) and without any further agitation for a period of time.
Step 230 is aimed primarily at providing the desaturase with the opportunity to carry out the desired conversion of saturated fatty acids in the cocoa butter to unsaturated fatty acids. As such, step 230 includes ensuring that the materials are maintained under conditions favorable for the conversion of during the time period. This includes maintaining the temperature of the mixture at a temperature not exceeding 93°F (34°C) so that the desaturase is not destroyed, and refraining from further agitation so as to not disrupt the desaturase acting on the saturated fatty acids. In some embodiments, the temperature of the mixture is maintained at about 93°F (34°C) for most of all of the time period.
Any manner of maintaining the temperature at about 93°F (34°C) can be used, such as through the periodic or continuation addition of a heat source to the mixture.

The period of time during which the mixture is held at the desired conditions in step 230 is generally not limited. In some embodiments, the period of time is any period of time necessary for conversion of the desired amount of saturated fatty acids to unsaturated fatty acids. In some embodiments, the mixture is held under the specific conditions for a period of time range from 24 to 48 hours, though more or less time may be used depending on the extent of conversion accomplished during the specified time period.

Upon completion of step 230, and more specifically, upon completion of the desired amount of desaturation of the cocoa butter, step 240 is carried out to eradicate the desaturase present in the mixture. Step 240 therefore generally involves heating the mixture to a temperature above 93°F (34°C) and at which the desaturase is killed, deactivated or otherwise destroyed and inhibited from further conversion of saturated fatty acids. In some embodiments, the mixture is heated to a temperature in the range of about 140°F (60°C). Any manner of heating the mixture to the elevated temperature can be used. Similarly, the time period during which the mixture is heated at the elevated temperature is generally not limited, provided that sufficient heat and time is applied in order to eradicate most or all of the desaturase. It should be noted that complete eradication of the desaturase may be preferable, but is not required.

In step 250, the mixture is filtered or otherwise processed in a manner that separates particulate from the mixture. Any manner of filtration or separation can be used provided that the separation method used separates a sufficient amount of particulate from the mixture. The particulate separated from the mixture generally includes, but is not limited to, the eradicated desaturase. In embodiments where step 240 does not eradicate all desaturase, the separation step may also remove active desaturase. In one non-limiting example, separation of particulate from the mixture is carried out by standard filtration techniques, such as vacuum filtration or centrifuge filtration. In some embodiments, the mixture is filtered to the point of meeting ISO 14/12/9 (to less than 1 micron filtration).

Referring back to Fig. 1, step 110 of desaturating the cocoa butter in method 100 is followed by step 120, in which the desaturated cocoa butter produced from step 110 is subjected to transesterification to thereby produce biodiesel. The process of transesterifying a vegetable oil, cooking oil, animal fat or the like in order to produce biodiesel is generally well known, and the transesterification process of step 110 as performed on the desaturated cocoa butter produced from step 110 can generally proceed in a manner similar or identical to previously known transesterification methods known for use in producing biodiesel. Generally speaking, the transesterification process entails reacting the fatty acid components of the desaturated cocoa butter with an alcohol, typically in the presence of a catalyst.

Thus, as described previously, the transesterification of step 120 generally requires the desaturated cocoa butter, an alcohol and a catalyst. The alcohol is typically methanol or ethanol, though other suitable alcohols can be used. The catalyst is typically a strong acid or a strong base. Non-limiting examples of a suitable catalyst is potassium hydroxide or sodium hydroxide.

In some embodiments, the transesterification process of step 120 begins by mixing together the catalyst and alcohol. The alcohol and catalyst are mixed until a fully homogenized solution is formed. Following formation of this mixture, the mixture can be combined with the desaturated cocoa butter produced in step 110. As noted previously, the manner in which the cocoa butter is desaturated produces a liquid desaturated cocoa butter, thus making the process of mixing the catalyst and alcohol with the desaturated cocoa butter much easier than if the cocoa butter was in a solid state. During the mixing of the desaturated cocoa butter with the alcohol and catalyst, heat may be applied to improve mixing and to begin the process of promoting the transesterification reaction. In such embodiments, it may be preferable to avoid heating the mixture to a temperature above 140°F (60°C) to thereby avoid scalding the cocoa butter.

Once combined, the mixture of catalyst, alcohol and desaturated cocoa butter may be mixed for a period of time to promote intimate mixing between the components and begin the process of promoting the transesterification reaction. The materials may be mixed for any suitable period of time provided that the materials are homogenously mixed. In some embodiments, the materials are mixed for a period of time in the range of from 15 to 20 minutes. In some embodiments, care is taken to not mix the materials for any longer than is required to create a homogenized mixture.

Following a suitable amount of mixing, the mixture of desaturated cocoa butter, catalyst and alcohol may be held for a period of time without further mixing or agitation, and with the application of heat to further promote the transesterification reaction. In some embodiments, the mixture is held for a period of time in the range of from 8-24 hours, and at a temperature in the range of 50 to 70°F (10 to 21°C). These conditions generally allow for the transesterification reactions to occur to the extent desired such that the cocoa butter is mostly or fully converted to biodiesel and glycol. These conditions also allow for the biodiesel and glycol products to separate, with the glycol component typically settling to the bottom of the product mixture.

After the desired period of time selected for desired reaction and settling, the glycerol can be separated from the biodiesel using any suitable separation methods. As noted previously, the glycerol may settle to the bottom of the product mixture, and therefore in some embodiments, the glycerol can be separated from the biodiesel via gravity drainage of the glycerol from the reaction vessel.

While not illustrated in Fig. 1, method 100 may further includes various washing and/or drying steps following the completion of step 130 in order to better condition the biodiesel for subsequent steps in method 100. Washing steps are generally aimed at removing soaps and other contaminants (e.g., residual methanol, potassium hydroxide, etc.) from the biodiesel. Any suitable washing techniques can be used provided the washing techniques remove undesired components from the biodiesel. In some embodiments, washing the biodiesel is carried out by either bubble washing or mist washing the biodiesel. The duration of the washing, the number of washing cycles, and which specific washing techniques are used are generally not limited. In some embodiments, both bubble washing and mist washing can be used to wash the biodiesel.

Drying steps that can be performed after the completion of step 140 (and after any optional washing steps carried out on the biodiesel) are generally aimed at reducing the water content of the biodiesel. It is desirable to reduce the water content of the biodiesel to avoid problems such as rusting or hydrolock. Any specific method of drying the biodiesel can be used. For example, in some embodiments, the biodiesel is heated to a temperature in the range of about 220°F (104°C), which results in water boiling off of the biodiesel.

In step 130 of method 100, urea clathration is performed on the biodiesel to thereby form clathrates in the biodiesel. Urea clathration generally includes adding urea and a solvent to the biodiesel produced in step 120 (and which may have been washed and dried as described previously) such that the urea forms into a lattice-like structure having saturated fatty acid methyl esters trapped therein. These lattice structures are solid masses also referred to as clathrates. Once formed, these clathrates can be readily removed from the biodiesel as discussed in greater detail below with respect to step 140. Once removed, the amount of unsaturated fatty acids in the biodiesel is reduced, which thereby further reduces the cloud point of the biodiesel.

In some embodiments, step 130 is performed by first mixing crystalized urea with a solvent to thereby dissolve the urea in the solvent and form a mixture. Any suitable solvent can be used to create this mixture with urea, provided the urea is capable of being dissolved in the solvent. In some embodiments, the solvent is an alcohol, such as methanol. The ratio of solvent to urea in the mixture is generally not limited, provided that a sufficient amount of solvent relative to the amount of urea is provided for full dissolution of urea in the solvent. In one example, the volumetric ratio of solvent to urea is 3:1. Once this mixture is prepared, the mixture is combined with the biodiesel produced in step 120. Once combined, the materials are mixed for a period of time to help begin the process of the formation of clathrates in the mixture. Any suitable period of time can be used for the mixing step and can be carried out using any suitable mixing methods and apparatus. In some embodiments, the mixing is carried out for about 10 minutes.

After the mixing step, the mixture of solvent, urea and biodiesel is held for a period of time without agitation to allow the clathration process to continue to progress. Any suitable amount of time needed for sufficient clathration to proceed can be used. In some embodiments, the mixture is held for about 12 hours.

Following the creation of clathrate masses in step 130, method 100 includes a step 140 of separating the clathrates from the biodiesel. Any suitable method of separating the clathrate masses from the biodiesel can be used, such as through filtration or draining clathrate masses from the mixture. The biodiesel can be subjected to multiple separation steps (e.g., multiple filtrations) and/or multiple separation techniques (e.g., press filtration and centrifugation filtration) in order to improve the amount of clathrate masses removed from the biodiesel. Because the clathrate masses include unsaturated fatty acids that were present in the biodiesel produced in step 130, the more complete the separation of the clathrates from the biodiesel, the more saturated fatty acid methyl esters are removed from the biodiesel. This correspondingly further improves the reduction in the cloud point of the biodiesel.

The method 100 illustrated in Fig. 1 and discussed in detail previously includes an initial step wherein cocoa butter is desaturated using a desaturase. However, it should be appreciated that other methods, not according to the invention, of desaturating cocoa butter can be used in the method described herein. With reference to Fig. 3, the desaturation of cocoa butter can take the form of one or more the illustrated methods. The first two exemplary methods, including adding an enzyme desaturase to melted cocoa butter or adding a cyanobacteria desaturase to melted cocoa butter, are in accordance with the invention. The third exemplary, not according to the invention, includes fractionating the cocoa butter to separate a specific fraction of the cocoa butter from the cocoa butter prior to transesterification. For example, fractionation can be carried out to specifically target a saturated and/or unsaturated fatty acid fraction of the cocoa butter. Any specific technique for fractionation can be carried out. The fractionation technique is fractional crystallization, a technique that is especially well suited for separating saturated and/or unsaturated fatty acids from the cocoa butter.

Benefits of the technology described herein include, but are not necessarily limited to, the following:
*Provides Reduced Cloud Point Biodiesel:* As discussed previously, most biodiesel fuels suffer from a cloud point that is too high for the biofuel to be used in colder climates. For example, as temperatures approach freezing, many biodiesel fuels suffer from a crystallization/solidification problem, wherein saturated and unsaturated fatty acid ester constituents within the biodiesel fuel crystalize/solidify. The crystalized/solidified constituents can clog fuel filters in vehicles and thereby cut off fuel supply to the engine. The technology described herein provides a biodiesel fuel that has a cloud point that may be as low as -61°F (-52°C), thus eliminating the cloud point problem described previously. As such, vehicles running on the biofuel produced by the methods described herein can be operated at practically any temperature that most vehicles will experience.
*Eliminates Need for Environmentally Unfriendly Additives and Blending Methods:* Prior methods used to attempt to lower the cloud point of biodiesel fuels include adding additives to the biodiesel and/or blending the biodiesel with traditional diesel fuel. Neither of these methods is environmentally friendly. Many additives that are used to lower the cloud point of biodiesel are expensive, contain registered carcinogens, and increase emissions and environmental pollutants expelled from a vehicle's exhaust line, such as nitrogen oxide and sulfur dioxide, both of which are known to cause acid rain. Blending requires a continued reliance on dirty fossil-based fuels and prevents the biodiesel from being considered a truly "clean" energy source. Neither additives nor blending are required for the biodiesel fuel produced by the method described herein, as the cloud point of such biodiesel fuel is sufficiently low as to not require additives or blending to further reduce the cloud point.
*Provides a Renewable Energy Source with Broad Applicability:* Because most biodiesel fuels suffer from a relatively high cloud point, the biodiesel is considered a renewable energy source with limited applicability. In contrast, the technology described herein produces a biodiesel with a substantially reduced cloud point such that the biodiesel fuel is usable across a broad range of temperatures without limitation. As a result, a practical and commercially feasible renewable energy source is provided
*Eliminates the Use of Environmentally Unfriendly Source Material In the Production of Biodiesel.* Most biodiesel is currently produced using soybean oil and palm oil as an initial source material. However, production of this source material has been and continues to be a major driver of deforestation of some of the world's most biodiverse forests, destroying the habitat of already endangered species. The use of cocoa butter as a source material for the production of biodiesel would avoid these problems, as the production of cocoa butter is not tied to deforestation.
*Opens Cocoa Butter as a Viable Source Material in the Production of Biodiesel:* The methods described herein make cocoa butter a viable source material for the production of biodiesel because the method overcomes the two main issues previously experienced with respect to using cocoa butter as a source material for the production of biodiesel: the solid nature of cocoa butter at room temperature making the material difficult to work with, and the high cloud point of biodiesel produced from cocoa butter. With respect to the solid nature of cocoa butter, the technology described herein includes steps that convert the cocoa butter from a fat (solid at room temperature) to a liquid, thus making the source material much easier to use in subsequent biodiesel production steps. With respect to the cloud point of biodiesel produced from cocoa butter, the dual step cold stabilization technology described herein produces a cocoa butter-derived biodiesel having a suitable low cloud point such that the resultant biodiesel is usable across almost all operating temperatures that would be expected for most vehicles. Without the technology described herein, cocoa butter produces a biodiesel fuel having a cloud point in the range of 59 to 62°F (15 to 17°C), thus making it an impractical fuel. In contrast, cocoa butter-derived biodiesel produced by the methods described herein has a cloud point as low as -61°F (-52°C).
*Uses Predominantly Reusable Materials In the Production of Biodiesel:* Almost all materials used in the biodiesel production methods described here are reusable, recycled, and/or recyclable materials. The cocoa butter source material itself is an industrial waste from the production of chocolate, and thus the method described herein provides a manner of using this otherwise waste material. The urea used in the clathration step is a reusable, naturally occurring compound produced by mammalian bodies, and can also be washed out of the clathrates formed in the disclosed methods and recovered at about 97% such that it can be reused in the disclosed methods. Methanol can be sourced from research university and labs, and may even be producible from, e.g., pallets used to deliver cocoa beans. All of the above works towards the described processes being a zero-waste process that reduces waste in other processes and may thereby drastically reduce the cost of production.
*Produces Biodiesel that Performs Comparably or Better Than Petroleum Diesel:* A common misconception regarding biofuels is that they lack in power output compared to a traditional petroleum diesel fuels such as No. 1 or No. 2 diesel, kerosene, Jet A, JP8, heating oil, or any other distillate fuel. However, power output of biodiesels prepared according to the methods described herein compare favorably to traditional petroleum diesel. This is due at least in part to the caloric density of cocoa butter being much higher than that of petroleum, which consists mainly of decomposed plant matter (primarily zooplankton and algae). Furthermore, biodiesel produced by the methods described herein is fully interchangeable with petroleum diesel. Both a modern Cummins diesel engine and a Mercedes diesel performance race vehicle have been run on 100% biodiesel produced from the methods described herein and have shown not only to match but to exceed traditional petroleum diesels in terms of economy, match in power output, as well as reduced emissions by more than 96%. This has also suggested that neither the engine nor the fuel system need modifications to run on the biodiesel produced by the methods described herein.
*Reduces or Eliminates Dependency on Fossil Fuels:* By producing an efficient and broadly applicable biodiesel, the methods described herein can reduce or eliminate the world's current dependency on fossil fuels as a source of energy. And the benefits of reducing the world's dependency on fossil fuels are many and well known, including eliminating conflict associated with fossil fuels, creating a cleaner environment, and diverting energy and resources away from extracting crude oil.

Unless otherwise indicated, all number or expressions, such as those expressing dimensions, physical characteristics, etc., used in the specification (other than the claims) are understood as modified in all instances by the term "approximately". At the very least, each numerical parameter recited in the specification or claims which is modified by the term "approximately" should at least be construed in light of the number of recited significant digits and by applying rounding techniques. Moreover, all ranges disclosed herein are to be understood to encompass and provide support for claims that recite any and all sub-ranges or any and all individual values subsumed therein. For example, a stated range of 1 to 10 should be considered to include and provide support for claims that recite any and all sub-ranges or individual values that are between and/or inclusive of the minimum value of 1 and the maximum value of 10; that is, all sub-ranges beginning with a minimum value of 1 or more and ending with a maximum value of 10 or less (e.g., 5.5 to 10, 2.34 to 3.56, and so forth) or any values from 1 to 10 (e.g., 3, 5.8, 9.9994, and so forth).

## Claims

1. A method of producing biodiesel from cocoa butter, comprising:
desaturating cocoa butter using a fatty acid desaturase to thereby produce a desaturated cocoa butter;
transesterifying the desaturated cocoa butter to thereby produce biodiesel;
performing urea clathration on the biodiesel to thereby form clathrates in the biodiesel; and
separating the clathrates from the biodiesel.

2. The method of claim 1, wherein the fatty acid desaturase comprises an enzyme fatty acid desaturase or a cyanobacteria fatty acid desaturase.

3. The method of claim 2, wherein the fatty acid desaturase comprises an enzyme fatty acid desaturase, and the enzyme fatty acid desaturase comprises cytochrome b5.

4. The method of claim 2, wherein the fatty acid desaturase comprises a cyanobacteria fatty acid desaturase, and the cyanobacterial fatty acid desaturase comprises a group 3y cyanobacteria.

5. The method of claim 1, wherein desaturating cocoa butter using a fatty acid desaturase to thereby produce a desaturated cocoa butter comprises:
melting the cocoa butter to a temperature not greater than about 93°F (34°C);
mixing together the melted cocoa butter and the fatty acid desaturase; and
holding the mixture of melted cocoa butter and fatty acid desaturase at a temperature not greater than 93°F (34°C) and without agitation for a period of time such that the fatty acid desaturase converts at least a portion of the saturated fatty acids in the melted cocoa butter to unsaturated fatty acids and thereby forms desaturated cocoa butter.

6. The method of claim 5, wherein the period of time is from 24 to 48 hours.

7. The method of claim 5, wherein desaturating cocoa butter using a fatty acid desaturase to thereby produce a desaturated cocoa butter further comprises:
heating the desaturated cocoa butter to a temperature above about 93°F (34°C); and
filtering the desaturated cocoa butter to remove particulate from the desaturated cocoa butter.

8. The method of claim 7, wherein heating the desaturated cocoa butter to a temperature above about 93°F (34°C) comprises heating the desaturated cocoa butter to a temperature of about 140°F (60°C).

9. The method of claim 1, wherein transesterifying the desaturated cocoa butter to thereby produce biodiesel comprises:
mixing a catalyst and an alcohol to form a homogenized solution;
mixing the homogenized solution with the desaturated cocoa butter to thereby produce a mixture of biodiesel and a glycerol byproduct; and
separating the glycerol byproduct from the biodiesel.

10. The method of claim 9, wherein the catalyst comprises potassium hydroxide or sodium hydroxide, and the alcohol comprises methanol or ethanol.

11. The method of claim 9, wherein separating the glycerol byproduct from the biodiesel comprises gravity draining the glycerol from the biodiesel.

12. The method of claim 1, wherein performing urea clathration on the biodiesel to thereby form clathrates in the biodiesel comprises:
preparing a mixture of urea and a solvent;
mixing the biodiesel with the mixture of urea and solvent; and
holding the mixture of biodiesel, urea and solvent for a period of time sufficient for the urea to bond to unsaturated fatty acids present in the biodiesel and thereby form clathrates.

13. The method of claim 12, wherein the solvent comprises methanol.

14. The method of claim 1, wherein separating clathrates from the biodiesel comprises centrifuge filtering the clathrates from the biodiesel.

15. The method of claim 1, further comprising, prior to performing urea clathration on the biodiesel:
washing the biofuel; and
drying the biofuel.

## Patentansprüche

1. Ein Verfahren zur Herstellung von Biodiesel aus Kakaobutter, das Folgendes umfasst:
Entsättigen von Kakaobutter mit Hilfe einer Fettsäure-Desaturase, um so eine entsättigte Kakaobutter herzustellen;
Umestern der entsättigten Kakaobutter zur Herstellung von Biodiesel;
Durchführen der Harnstoff-Clathration mit dem Biodiesel, um dadurch Clathrate im Biodiesel zu bilden, und
Trennen der Clathrate vom Biodiesel.

2. Das Verfahren nach Anspruch 1, wobei die Fettsäure-Desaturase eine Enzym-Fettsäure-Desaturase oder eine Cyanobakterien-Fettsäure-Desaturase umfasst.

3. Das Verfahren nach Anspruch 2, wobei die Fettsäure-Desaturase eine Enzym-Fettsäure-Desaturase umfasst und die Enzym-Fettsäure-Desaturase Cytochrom b5 umfasst.

4. Das Verfahren nach Anspruch 2, wobei die Fettsäure-Desaturase eine Cyanobakterien-Fettsäure-Desaturase umfasst und die cyanobakterielle Fettsäure-Desaturase ein Cyanobakterium der Gruppe 3y umfasst.

5. Das Verfahren nach Anspruch 1, wobei das Entsättigen von Kakaobutter unter Verwendung einer Fettsäure-Desaturase, um dadurch eine entsättigte Kakaobutter herzustellen, Folgendes umfasst:
Schmelzen der Kakaobutter auf eine Temperatur von nicht mehr als 93 °F (34 °C);
Mischen der geschmolzenen Kakaobutter und der Fettsäure-Desaturase und
Halten der Mischung aus geschmolzener Kakaobutter und Fettsäure-Desaturase auf einer Temperatur von nicht mehr als 93 °F (34°C) und ohne Rühren für eine solche Zeitspanne, dass die Fettsäure-Desaturase mindestens einen Teil der gesättigten Fettsäuren in der geschmolzenen Kakaobutter in ungesättigte Fettsäuren umwandelt und dadurch entsättigte Kakaobutter bildet.

6. Das Verfahren nach Anspruch 5, wobei die Zeitspanne zwischen 24 und 48 Stunden beträgt.

7. Das Verfahren nach Anspruch 5, wobei das Entsättigen von Kakaobutter unter Verwendung einer Fettsäure-Desaturase, um dadurch eine entsättigte Kakaobutter herzustellen, weiterhin Folgendes umfasst:
Erhitzen der entsättigten Kakaobutter auf eine Temperatur über etwa 93 °F (34°C) und
Filtern der entsättigten Kakaobutter, um Partikel aus der entsättigten Kakaobutter zu entfernen.

8. Das Verfahren nach Anspruch 7, wobei das Erhitzen der entsättigten Kakaobutter auf eine Temperatur über etwa 93 °F (34 °C) das Erhitzen der entsättigten Kakaobutter auf eine Temperatur von etwa 140 °F (60 °C) umfasst.

9. Das Verfahren nach Anspruch 1, wobei das Umestern der entsättigten Kakaobutter zur Herstellung von Biodiesel Folgendes umfasst:
Mischen eines Katalysators und eines Alkohols zur Bildung einer homogenisierten Lösung;
Mischen der homogenisierten Lösung mit der entsättigten Kakaobutter, um so eine Mischung aus Biodiesel und einem Glycerin-Nebenprodukt herzustellen, und
Trennen des Glycerin-Nebenprodukts vom Biodiesel.

10. Das Verfahren nach Anspruch 9, wobei der Katalysator Kaliumhydroxid oder Natriumhydroxid umfasst und der Alkohol Methanol oder Ethanol umfasst.

11. Das Verfahren nach Anspruch 9, wobei das Trennen des Glycerin-Nebenprodukts vom Biodiesel die Schwerkraftentleerung des Glycerins aus dem Biodiesel umfasst.

12. Das Verfahren nach Anspruch 1, wobei das Durchführen der Harnstoff-Clathration mit dem Biodiesel, um dadurch Clathrate in dem Biodiesel zu bilden, Folgendes umfasst:
Herstellen einer Mischung aus Harnstoff und einem Lösungsmittel;
Mischen des Biodiesels mit der Mischung aus Harnstoff und Lösungsmittel und
Halten der Mischung aus Biodiesel, Harnstoff und Lösungsmittel für einen Zeitraum, der ausreicht, damit sich der Harnstoff an die im Biodiesel vorhandenen ungesättigten Fettsäuren bindet und dadurch Clathrate bildet.

13. Das Verfahren nach Anspruch 12, wobei das Lösungsmittel Methanol umfasst.

14. Das Verfahren nach Anspruch 1, wobei das Trennen von Clathraten vom Biodiesel die Zentrifugalfilterung der Clathrate aus dem Biodiesel umfasst.

15. Das Verfahren nach Anspruch 1, das ferner vor der Durchführung der Harnstoff-Clathration mit dem Biodiesel Folgendes umfasst:
Waschen des Biokraftstoffs und
Trocknen des Biokraftstoffs.

## Revendications

1. Un procédé de production de biodiesel à partir de beurre de cacao, consistant à :
désaturer le beurre de cacao à l'aide d'une désaturase d'acides gras pour obtenir un beurre de cacao désaturé ;
transestérifier le beurre de cacao désaturé pour produire du biodiesel ;
effectuer une clathration à l'urée sur le biodiesel pour former des clathrates dans le biodiesel ; et à
séparer les clathrates du biodiesel.

2. Le procédé de la revendication 1, dans lequel la désaturase d'acides gras comprend une enzyme désaturase d'acides gras ou une désaturase d'acides gras de cyanobactéries.

3. Le procédé de la revendication 2, dans lequel la désaturase d'acides gras comprend une enzyme désaturase d'acides gras et l'enzyme désaturase d'acides gras comprend le cytochrome b5.

4. Le procédé de la revendication 2, dans lequel la désaturase d'acides gras comprend une désaturase d'acides gras de cyanobactéries et la désaturase d'acides gras de cyanobactéries comprend une cyanobactérie du groupe 3y.

5. Le procédé de la revendication 1, dans lequel la désaturation du beurre de cacao à l'aide d'une désaturase d'acides gras visant à produire un beurre de cacao désaturé consiste à :
faire fondre le beurre de cacao à une température ne dépassant pas environ 34°C (93°F) ;
mélanger le beurre de cacao fondu et la désaturase d'acides gras ; et à
maintenir le mélange de beurre de cacao fondu et de désaturase d'acides gras à une température ne dépassant pas 34°C (93°F) et sans agitation pendant une période suffisante pour que la désaturase d'acides gras transforme au moins une partie des acides gras saturés du beurre de cacao fondu en acides gras insaturés et forme ainsi du beurre de cacao désaturé.

6. Le procédé de la revendication 5, dans lequel la période est comprise entre 24 et 48 heures.

7. Le procédé de la revendication 5, dans lequel la désaturation du beurre de cacao à l'aide d'une désaturase d'acides gras visant à produire un beurre de cacao désaturé consiste en outre à :
chauffer le beurre de cacao désaturé à une température supérieure à environ 34°C (93°F) ; et à
filtrer le beurre de cacao désaturé afin d'éliminer les particules présentes dans celui-ci.

8. Le procédé de la revendication 7, dans lequel le chauffage du beurre de cacao désaturé à une température supérieure à environ 34°C (93°F) consiste à chauffer le beurre de cacao désaturé à une température d'environ 60°C (140°F).

9. Le procédé de la revendication 1, dans lequel la transestérification du beurre de cacao désaturé visant à produire du biodiesel consiste à :
mélanger un catalyseur et un alcool pour former une solution homogénéisée ;
mélanger la solution homogénéisée avec le beurre de cacao désaturé pour produire un mélange de biodiesel et d'un sous-produit glycérol ; et à
séparer le sous-produit glycérol du biodiesel.

10. Le procédé de la revendication 9, dans lequel le catalyseur comprend de l'hydroxyde de potassium ou de l'hydroxyde de sodium et l'alcool comprend du méthanol ou de l'éthanol.

11. Le procédé de la revendication 9, dans lequel la séparation du sous-produit glycérol du biodiesel consiste à drainer par gravité le glycérol du biodiesel.

12. Le procédé de la revendication 1, dans lequel la clathration à l'urée sur le biodiesel visant à former des clathrates dans le biodiesel consiste à :
préparer un mélange d'urée et de solvant ;
mélanger le biodiesel avec le mélange d'urée et de solvant ; et à
maintenir le mélange de biodiesel, d'urée et de solvant pendant une période suffisante pour que l'urée se lie aux acides gras insaturés présents dans le biodiesel et forme ainsi des clathrates.

13. Le procédé de la revendication 12, dans lequel le solvant comprend du méthanol.

14. Le procédé de la revendication 1, dans lequel la séparation des clathrates du biodiesel consiste à filtrer par centrifugation les clathrates du biodiesel.

15. Le procédé de la revendication 1, consistant en outre, avant d'effectuer la clathration à l'urée sur le biodiesel, à :
laver le biocarburant ; et à
sécher le biocarburant.
